# EUROPEAN PATENT SPECIFICATION

(11) **EP 1 471 995 B1**
(45) Date of publication and mention of the grant of the patent: **23.07.2008**
(21) Application number: 03708081.9
(22) Date of filing: 03.02.2003
(51) Int. Cl.: B01J 13/18, B01J 13/20, A61K 8/11

(54) **ENCAPSULATION PROCESS AND ENCAPSULATED COMPOSITIONS**
UMHÜLLUNGSVERFAHREN UND GEKAPSELTE ZUSAMMENSETZUNGEN
PROCEDE D'ENCAPSULATION ET COMPOSITIONS ENCAPSULEES

(30) Priority: 07.02.2002 GB 0202853
(43) Date of publication of application: 03.11.2004
(73) Proprietor: Dow Corning Corporation, Midland, Michigan 48611 (US)
(72) Inventor: MARTEAUX, Leon, B-1160 Brussels (BE); GIRBOUX, Anne-Lise, B-1420 Braine l'Alleud (BE); LABROSSE, Arnaud, B-7170 Manage (BE); POLSTER-RENARD, Catherine, B-7830 Hellebecq (BE); VAN REETH, Isabelle, B-1315 Incourt (BE)
(74) Representative: Davies, Peter Vaughan
(86) International application number: PCT/EP2003/001071
(87) International publication number: WO 2003/066209

(56) References cited:
- WO-A-01/80823
- DE-A- 19 954 772
- US-A- 4 169 069
- US-B1- 6 238 650

## Description

### FIELD OF THE INVENTION

This invention relates to a process for encapsulating materials such as cosmetic, chemical or pharmaceutical active material compositions and to the encapsulated compositions which can be formed thereby. It is of particular use in encapsulating sunscreen active materials and can also be used for other cosmetic actives, such as perfumes, for other chemical materials and for pharmaceutical active materials.

In some personal care products, the cosmetic active material may be present in high amounts. For example, a SPF (sun protection factor) 15 sun cream or lotion may contain over 5% by weight sunscreen active materials (UV blockers). Moreover, some cosmetic active materials, including UV blockers, can be skin irritants or should not be absorbed through the skin. There is a need for products which prevent or inhibit skin contact by such a cosmetic active material.

### BACKGROUND TO THE INVENTION

JP-A-2-2867 describes sunscreen benzophenone derivatives encapsulated in fine spherical silica particles. The sunscreen is dissolved in aqueous alkali metal silicate solution and is emulsified in an organic non-solvent to form a water-in-oil emulsion. The emulsion is acidified to form a water-insoluble precipitate of sunscreen encapsulated in silica. The process of JP-A-2-2867 is suitable for hydrophilic sunscreen active materials, but most sunscreen active materials are lipophilic.

WO-A-98/31333 describes sunscreen-doped sol-gel materials and a method for their preparation comprising condensation polymerising a metal or semi-metal alkoxide or ester in the presence of at least one sunscreen ingredient, resulting in the entrapment of the sunscreen ingredients within the formed sol-gel matrix.

US-A-6303149 describes a process for preparing sol-gel microcapsules loaded with functional molecules by emulsifying sol-gel precursors and the functional molecules in an aqueous solution, and mixing the emulsion with an acidic, neutral or basic aqueous solution to obtain a suspension of microcapsules. US-A-6238650 describes a sunscreen composition comprising at least one sunscreen active ingredient and a cosmetically acceptable vehicle, wherein said sunscreen active ingredient is in the form of sol-gel microcapsules containing at least one sunscreen compound. The sol-gel microcapsules are prepared by the method disclosed in US-A-6303149.

EP-A-281034 describes a perfume encapsulated and/or clathrated in a matrix of inorganic polymer prepared from a metal alkoxide such as tetraethyl orthosilicate (TEOS). An aqueous dispersion or solution of perfume and TEOS is treated with an acid catalyst to cause hydrolysis, then with a base catalyst to cause polymerisation to a gel.

EP-A-941761 describes a process for preparing microcapsules with an organopolysiloxane shell and a core material, in which the shell is formed in situ by hydrolysis and polycondensation of an organosilane and/or a condensation product thereof having at most 4 silicon atoms. JP-51-78995-A describes dispersing a silyl-treated pigment with TEOS in acetone and adding to ammoniacal aqueous ethanol with stirring to form a micropowder of particles having a pigment core.

EP-A-934773 describes microcapsules whose capsule wall comprises organopolysiloxane synthesised by polycondensing a compound of the formula RₙSi(OH)ₘY₍₄₋ₘ₋ₙ₎ where m=1-4; n=0-3; R represents an organic group with a C atom directly bonded to a SI atom; and Y is an alkoxy group, H or a siloxy group.

WO-A-00/71084 describes preparing a sunscreen composition with improved photostability that contains at least two sunscreen actives which are photo-unstable when formulated together by microencapsulating at least one of the actives and adding other components of the sunscreen composition.

WO-A-01/80823 describes a therapeutic or cosmetic composition comprising microcapsules of diameter 0.1-100µ having a core-shell structure. The core includes at least one active. The shell comprises an inorganic polymer obtained by a sol-gel process, and releases the active after topical application.

There is a need for cosmetic active materials, particularly sunscreens, in a form in which the active material is inhibited from skin contact but which contains a high proportion of the active material and can readily be incorporated into a cosmetic preparation such as a lotion or cream.

### SUMMARY OF THE INVENTION

According to one aspect of the present invention, a process for encapsulating a lipophilic cosmetic, chemical, biological or pharmaceutical active material composition is characterised in that a water reactive silicon compound comprising tetraalkoxysilane is added to an aqueous emulsion of the active material composition having a positive zeta-potential, whereby the tetraalkoxysilane condenses and polymerises at the interface of the droplets in the emulsion to form microcapsules having a core of the active material composition surrounded by a shell of silicon-based network polymer. We use the term 'emulsion' to mean a liquid in liquid dispersion and 'suspension' to mean a solid in liquid dispersion.

The invention also includes an encapsulated cosmetic, chemical, biological or pharmaceutical active material composition, characterised in that the encapsulated composition comprises microcapsules of a lipophilic cosmetic, chemical, biological or pharmaceutical active material composition encapsulated within a shell of the emulsion polymerisation product of a tetraalkoxysilane.

The invention also includes a process for the preparation of an encapsulated lipophilic cosmetic, chemical, biological or pharmaceutical active material composition, characterised in that an aqueous emulsion of the active material composition is mixed with a water-reactive silicon compound, thereby forming a suspension of microcapsules having a core of the active material composition and a shell of silicon-based network polymer, and the microcapsules are post-treated with a water-reactive metal alkoxy or acyloxy compound.

### DETAILED DESCRIPTION OF THE INVENTION

The tetraalkoxysilane such as tetraethoxysilane (TEOS) can be used in monomeric form or as a liquid partial condensate. The tetraalkoxysilane can be used in conjunction with one or more other water-reactive silicon compound having at least two, preferably at least 3, Si-OH groups or hydrolysable groups bonded to silicon, for example an alkyltrialkoxysilane such as methyltrimethoxysilane or a liquid condensate of an alkyltrialkoxysilane. Hydrolysable groups can for example be alkoxy or acyloxy groups bonded to silicon. The water reactive silicon compound can for example comprise 75-100% by weight tetraalkoxysilane and 0-25% trialkoxysilane. The alkyl and alkoxy groups in the tetraalkoxysilanes or other silanes preferably contain 1 to 4 carbon atoms, most preferably 1 or 2 carbon atoms. The tetraalkoxysilane, and other water-reactive silicon compound if used, hydrolyses and condenses to form a network polymer, that is a 3-dimensional network of silicon-based material, around the emulsified droplets of the lipophilic active material composition. The water-reactive silicon compound preferably consists of at least 75%, and most preferably 90-100% tetraalkoxysilane. We have found that a tetraalkoxysilane is the most effective silicon compound for forming impermeable microcapsules, forming a 3-dimensional network consisting substantially of SiO_{4/2} units.

The lipophilic cosmetic, chemical, biological or pharmaceutical active material composition is a liquid at the time it is emulsified and usually is liquid at ambient temperature. It can be an undiluted liquid active material or can be a solution of an active material in a lipophilic solvent, preferably a non-volatile solvent, or a water-in-oil or oil-in-water-in-oil emulsion, or a lipophilic suspension. Solid active materials can be melted before being emulsified if their melting temperature is significantly below 100oC.

The active material can for example be a sunscreen. Sunscreen active compounds which are used in the invention can for example be UV-B blockers such as 2-ethylhexyl methoxycinnamate, generally known as octyl methoxycinnamate or UV-A blockers such as butylmethoxydibenzoylmethane known as avobenzone. Mixtures of sunscreen compounds can be used, for example a mixture of octyl methoxycinnamate with octocrylene, although octyl methoxycinnamate and avobenzone are known to be photolytically unstable when mixed in high concentrations and should preferably not be encapsulated together. Octyl methoxycinnamate is liquid and can be used undiluted. Butylmethoxydibenzoylmethane is a solid which can be dissolved in an inert lipophilic liquid.

Sunscreen active compounds can perform their function of screening out harmful UV radiation while they are encapsulated. The silicon-based polymer forming the shell of the microcapsules does not absorb UV and has no negative impact on the sunscreen efficiency, and may potentially improve the protection against photodegradation. An encapsulated sunscreen according to the invention preferably has a substantially impermeable silicon-based polymer shell.

Another type of cosmetic active material which can be encapsulated is a perfume. In this case the active material composition which is mixed with the water reactive silicon compound may comprise a diluent as well as the active perfume or fragrance compounds. The diluent is preferably odourless and non-volatile and can for example be a non-reactive polydiorganosiloxane or a nonvolatile liquid hydrocarbon or ester. When encapsulating perfume, a permanent shell is not required. The shell may be breakable, for example brittle, so that the microcapsules are broken under the application of shear, releasing the perfume. Larger particles of diameter at least 10µm, for example 50µm or above, are more readily breakable than smaller particles.

Other examples of active materials which may be encapsulated are UV absorbers for use in coatings, paints, plastics materials, sealants or textile finishes to improve weatherability and resist fading (such UV absorber compositions are preferably encapsulated in an impermeable silicon-based polymer shell), pharmaceuticals or sensitive chemical materials. Pharmaceuticals and related health products such as vitamins can be encapsulated in a silicon-based polymer shell which is broken down in the body after ingestion of the pharmaceutical. Biological (including biochemical) materials such as proteins, enzymes and cells can similarly be encapsulated. Radioactive material can be encapsulated for cancer treatment. Water insoluble liquid chemical materials can be protected, for example during storage or transport. Encapsulation can alternatively be used to modify the surface properties, optical properties or feel and taste of any core material.

The lipophilic active material composition is emulsified in an aqueous medium with the aid of a surfactant. The particle size of the emulsion of active material composition is generally in the range 0.01 to 500, preferably 0,1 to 50 micrometres. The emulsion can alternatively be a microemulsion of particle size 10-150 nm. The surfactant is a cationic or amphoteric surfactant, which readily forms an emulsion of positive zeta-potential. We have found that a positive zeta-potential promotes condensation and polymerisation of the tetraalkoxysilane at the interface of the emulsified droplets of the lipophilic active material composition, leading to more impervious microcapsules. Nonionic surfactants can be used; for example the cationic or amphoteric surfactant can be mixed with up to an equal weight of nonionic surfactant.

The concentration of surfactant in the aqueous emulsion of lipophilic active material can be between 0.01 and 10% by weight, but is preferably at least 0.02% and below 2%, most preferably 0.05 to 1.5% by weight of the emulsion, particularly 0.2-1.0%. We have found in general that the use of low levels of surfactant during emulsification of the lipophilic active material and reaction with the alkoxysilane leads to microcapsules which are more resistant to diffusion or leaching of the lipophilic active material from the microcapsules. Subsequent addition of surfactant to the suspension of microcapsules has less or no effect on diffusion or leaching of the lipophilic active material from the microcapsules.

The weight ratio of oil phase to aqueous phase in the emulsion can generally be between 40:1 and 1:50, although the higher proportions of aqueous phase are economically disadvantageous particularly when forming an emulsion of microcapsules. Usually the weight ratio of oil phase to aqueous phase is between 2:1 and 1:3. If the active material composition is highly viscous, a phase inversion process can be used in which the oil phase is mixed with surfactant and a small amount of water, for example 2.5 to 10% by weight based on the oil phase, forming a water-in-oil emulsion which inverts to an oil-in-water emulsion as it is sheared. Further water can then be added to dilute the emulsion to the required concentration.

Examples of cationic surfactants include quaternary ammonium hydroxides such as octyl trimethyl ammonium hydroxide, dodecyl trimethyl ammonium hydroxide, hexadecyl trimethyl ammonium hydroxide, octyl dimethyl benzyl ammonium hydroxide, decyl dimethyl benzyl ammonium hydroxide, didodecyl dimethyl ammonium hydroxide, dioctadecyl dimethyl ammonium hydroxide, tallow trimethyl ammonium hydroxide and coco trimethyl ammonium hydroxide as well as corresponding salts of these materials, fatty amines and fatty acid amides and their derivatives, basic pyridinium compounds, quaternary ammonium bases of benzimidazolines and polypropanolpolyethanol amines. Cationic emulsions of microcapsules have increased deposition of the microcapsules from the emulsion and increased substantivity on both hair and skin.

Examples of suitable amphoteric surfactants include cocamidopropyl betaine, cocamidopropyl hydroxysulfate, cocobetaine, sodium cocoamidoacetate, cocodimethyl betaine, N-coco-3-aminobutyric acid and imidazolinium carboxyl compounds.

The above surfactants may be used individually or in combination.

Examples of non-ionic surfactants include polyoxyalkylene alkyl ethers such as polyethylene glycol long chain (12-14C) alkyl ether, polyoxyalkylene sorbitan ethers, polyoxyalkylene alkoxylate esters, polyoxyalkylene alkylphenol ethers, ethylene glycol propylene glycol copolymers, polyvinyl alcohol and alkylpolysaccharides, for example materials of the structure R¹-O-(R²O)ₘ-(G)ₙ wherein R¹ represents a linear or branched alkyl group, a linear or branched alkenyl group or an alkylphenyl group, R² represent an alkylene group, G represents a reduced sugar, m denotes 0 or a positive integer and n represent a positive integer as described in US Patent 5,035,832.

The continuous phase of the emulsion can be a mixture of water with a water-miscible organic solvent such as an alcohol or lactam provided that the continuous phase is not miscible with the lipophilic active material. The particle size of the emulsion of lipophilic active material can be reduced before addition of the water-reactive silicon compound, for example in an apparatus applying increased shear such as a homogeniser or microfluidiser, or a sonolator (ultrasonic mixer), producing an emulsion of microcapsules of particle size 100-1000 nm, most preferably between 200 nm and 500 nm. The emulsion can alternatively be prepared by phase inversion.

The particle size of the microcapsules produced generally corresponds to the particle size of the starting emulsion and can for example be in the range 0.01-500 µm, most preferably 200 nm to 10 µm. For some uses, microcapsules of particle size 1-500 µm, particularly up to 50 or 100 µm, may be preferred. If an emulsion of this particle size is required, the aqueous phase of the emulsion preferably contains a thickener, for example polyvinylpyrrolidone, polyvinyl alcohol, bentonite clay, a cellulose derivative, particularly a cellulose ether such as sodium carboxymethylcellulose, a lightly crosslinked acrylic polymer, modified starch, an alginate or xanthan gum, to inhibit settling of the microcapsules from the emulsion during formation or subsequently. The thickener is added to the emulsion before addition of the tetraalkoxysilane. We have found that addition of polyvinylpyrrolidone to the emulsion before addition of the tetraalkoxysilane promotes formation of microcapsules more resistant to diffusion of the lipophilic material from the microcapsules for most particle sizes of the microcapsules.

The tetraalkoxysilane, and other water reactive silicon compound if used, can be added to the emulsion of active material composition as an undiluted liquid or as a solution in an organic solvent or in an emulsion form. The tetraalkoxysilane and the emulsion are generally mixed under shear during addition and subsequently during condensation to form the silicon-based polymer shell on the surface of the emulsified droplets. Mixing can for example be by stirring, but it is preferred that the emulsion and the tetraalkoxysilane are subjected to high shear, for example in a mixer of the rotor and stator type such as a Silberson (trade mark) mixer, either during addition of the tetraalkoxysilane or after addition of the tetraalkoxysilane and before formation of microcapsules is complete. High shear mixing immediately after addition of the tetraalkoxysilane is preferred. This leads to microcapsules of reduced particle size and appears to promote polymerisation of substantially all the tetraalkoxysilane at the interface of the emulsion droplets.

The condensation reaction can be conducted at acidic, neutral or basic pH. The condensation reaction is generally carried out at ambient temperature and pressure, but can be carried out at increased temperature, for example up to 95oC, and increased or decreased pressure, for example under vacuum to strip the volatile alcohol produced during the condensation reaction. The weight ratio of active material composition to water reactive silicon compound is preferably at least 0.5:1 and in many cases may be at least 1.5:1, for example 2:1 to 9:1. Smaller microcapsules, for example those formed from a microemulsion, generally have a lower ratio of active material composition to water reactive silicon compound.

A catalyst for hydrolysis and/or condensation of the water reactive silicon compound to form the silicon-based polymer may be used. The catalyst is preferably an oil soluble organic metal compound, for example an organic tin compound, particularly an organotin compound such as a diorganotin diester, for example dimethyl tin di(neodecanoate), dibutyl tin dilaurate or dibutyl tin diacetate, or alternatively a tin carboxylate such as stannous octoate, or an organic titanium compound such as tetrabutyl titanate. An organotin catalyst can for example be used at 0.05 to 2% by weight based on the water reactive silicon compound. An organotin catalyst has the advantage of effective catalysis at neutral pH. A catalyst is most preferably mixed with the lipophilic cosmetic, chemical or pharmaceutical active material composition before it is emulsified, since this promotes condensation of the water reactive silicon compound at the surface of the emulsified lipophilic droplets. A catalyst can alternatively be added to the emulsion before the addition of the water-reactive silicon compound, or simultaneously with the water-reactive silicon compound, or after the addition of the water-reactive silicon compound to harden and make more impervious the shell of silicon-based polymer which has been formed. Encapsulation can however be achieved without catalyst, and we have found in some cases that microcapsules formed with a low level of catalyst or no catalyst are more resistant to diffusion or leaching of the lipophilic active material from the microcapsules. The catalyst, when used, can be added undiluted, or as a solution in an organic solvent such as a hydrocarbon, alcohol or ketone, or as a mutiphasic system such as an emulsion or suspension.

The product of hydrolysis and condensation of the water reactive silicon compound is an aqueous suspension of microcapsules. The aqueous continuous phase can contain water miscible organic solvent; for example it usually contains an alcohol such as ethanol generated by hydrolysis of Si-bonded alkoxy groups. It may be advantageous to use the suspension of microcapsules in a water based preparation, for example a cosmetic, chemical or pharmaceutical product without separating the microcapsules from the suspension. In particular, a suspension of encapsulated sunscreen can be incorporated direct into a sunscreen lotion or cream or can even be used itself as a sunscreen lotion. The suspension of encapsulated sunscreen can be used in conjunction with other sunscreens, if desired. For example, an encapsulated UV-B absorber such as octyl methoxycinnamate can be formulated with a UV-A absorber such as avobenzone and optionally with other sunscreens. The UV-A absorber in such a formulation can be free or encapsulated.

For many uses it may be preferred to recover the microcapsules from suspension, for example for subsequent dispersion in a different medium. An encapsulated sunscreen can for example be dispersed in a water based cosmetic preparation, preferably in such a proportion that the content of sunscreen in the cosmetic preparation is 0.1 to 10% by weight. Alternatively the microcapsules can be redispersed in an organic solvent, optionally with additives such as surfactant and/or polymer. Recovery of the microcapsules can be achieved by any known liquid removal technique, for example by spray drying, spray chilling, filtering, oven drying or lyophilisation.

Alternative uses of encapsulated sunscreens according to the invention are in fabric treatment, for example the suspension of microcapsules or the separated microcapsules can be incorporated in a fabric softener to inhibit subsequent colour fading of the fabric, or in plastics compositions or coatings which are designed to be exposed to sunlight or UV light in use.

The encapsulated product can be post-treated with a water-reactive metal alkoxy or acyloxy compound. The metal compound should be gradually hydrolysed in water rather than immediately reacting with water; compounds of Group IVB, IVA or VA of the Periodic Table are suitable such as compounds of silicon, titanium, zirconium or vanadium. The water-reactive metal alkoxy or acyloxy compound can for example harden the shell of the microcapsules and/or make them more impermeable. The reactive metal alkoxy or acyloxy compound can for example be an alkoxysilane or acyloxysilane, particularly a trialkoxysilane such as methyl triethoxy silane or isobutyl triethoxy silane, or a silane having Si-H functionality such as tris(dimethylhydrogensilyloxy) n-octyl silane, or alternatively a titanium alkoxide (alkyl titanate).

The reactive metal alkoxy or acyloxy compound can have an organic functional group to promote adhesion to substrates, especially textile substrates, for example 3-methacryloxypropyl trimethoxy silane, 3-aminopropyl triethoxysilane, 3-aminopropyl trimethoxy silane, 3-glycidoxypropyl trimethoxy silane and 3-(2-aminoethylamino)propyl trimethoxy silane. The microcapsules can be post-treated with a reactive metal alkoxy or acyloxy compounds, e.g. an alkoxysilane to change their physical and/or chemical properties, for example by making the capsule surface more hydrophobic or more hydrophilic. For example, the microcapsule surface can be made more hydrophobic by reaction with a silane having a long chain alkyl group such as octyl triethoxy silane. As an alternative to chemical reaction the microcapsules can be coated with a material which alters their surface properties. The surface treatment can be carried out on the microcapsules in suspension or on the separated solid microcapsules.

The microcapsules according to the invention inhibit diffusion or leaching of the lipophilic cosmetic, chemical, biological or pharmaceutical active material from the microcapsules. When encapsulating sunscreen, for example, it is preferred that the rate of diffusion or leaching is as low as possible. For other lipophilic active materials a controlled rate of release may be preferred, and this can be achieved by adjusting the level of surfactant, the level of tetraalkoxysilane and optionally of trialkoxysilane, the particle size and the level of catalyst.

Microcapsules according to the invention containing cosmetic active material compositions, including sunscreens, have good skin adhesion. The microcapsules minimise contact between the sunscreen and the skin, resulting in decreased penetration and consequently less potential irritation and allergy. The emulsion of microcapsules can have a high concentration of sunscreen (high pay load) compared to an aqueous dispersion of sunscreen, increasing the ease of use of lipophilic sunscreens in surfactant based product and allowing the sunscreen preparation to have a very liquid product form, which may be sprayable. Encapsulation eliminates the greasy feel associated with lipophilic sunscreens, increasing the acceptability and use in skin care products. The microcapsule does not affect the photostability of the encapsulated sunscreen. Exposure for 20 minutes to irradiation by a 19 mW/cm² UV A lamp and a 0.6 mW/cm² UV B lamp (equivalent to 1 hour sunshine as described in WO01/24762) gives less than 10% reduction in the sun protection factor of an encapsulated octyl methoxycinnamate composition. The silicon-based polymer forming the shell of the microcapsules generally remains water insoluble even in the presence of surfactant, so that the encapsulated cosmetic active can be used in water based toiletry preparations including surfactant based products such as hair shampoo, conditioner or colourant, textile softener, detergent or shower gel.

The invention is illustrated by the following Examples in which parts and percentages are by weight:

### EXAMPLES

### Example 1

41.33g octylmethoxycinnamate (OMC, a UV-B sunscreen oil) was emulsified in 53.1g water containing 1.64g Volpo L3 (Trade Mark) nonionic polyethylene glycol lauryl ether surfactant and 3.66g Arquad 16-29 (Trade Mark) cetyl trimethyl ammonium chloride cationic surfactant. The coarse emulsion was passed twice through a "Rannie Mini Lab 8.30 H" homogeniser operating at 950 bars. 17.71g TEOS was added to the emulsion while stirring to form a coarse emulsion of microcapsules. Microcapsules of median diameter 372nm were produced in suspension. When the microcapsules were tested using a leaching procedure to extract non-encapsulated oil, the degree of encapsulation was 97%.

### Example 2

Example 1 was repeated with the addition of 0.236g dimethyltin dineodecanoate catalyst just after the TEOS. Microcapsules of median diameter 372nm were produced with an encapsulation yield of 89%. The suspension of microcapsules had an OMC content of 35.1% and the separated microcapsules had an OMC content of 89%.

In vitro SPF (sun protection factor) measurements together with sensory evaluation have been conducted on the suspension of microcapsules in comparison to an emulsion containing the same level of OMC not encapsulated. The samples were applied to a substrate called " Mimskin(R) and SPF measurements were made using a Labsphere UV-1000 SPF Analyser at 25°C. The results are reported in Table 1 below

**Table 1**

| | In-vitro SPF |
|---|---|
| Emulsion with non encapsulated OMC | 7.41 |
| Encapsulated OMC of Example 2 | 24.4 |

Table 1 shows that the encapsulation does not have any negative impact on the sunscreen efficiency and could even have a positive impact.

Sensory evaluation by a triangular test method demonstrated a decrease of the greasy feel of the sunscreen.

The resistance of the encapsulated sunscreens of Examples 1 and 2 to leaching of OMC from the microcapsules was tested by an aggressive method in which the suspension of microcapsules was dispersed at 1% in a paraffinic solvent and the OMC content of the paraffin was measured by UV spectroscopy at different time intervals after dispersion. The concentration of OMC in the paraffin was found to increase in a linear manner with time (zero order delivery of OMC). In this test, 47% of the OMC was extracted from the microcapsules of Example 1 after 24 hours and 71 % of the OMC was extracted from the microcapsules of Example 2 after 24 hours. Without encapsulation, over 80% of the OMC is extracted from the emulsion of OMC in 4 hours. By comparison, when the Arquad 16-29 cationic surfactant was replaced by an anionic surfactant, substantially all the OMC was extracted from the capsules in 4 hours.

### Example 3

Example 1 was repeated except that 0.236g dimethyltin dineodecanoate catalyst was mixed into the OMC before it was emulsified, and only 46.6g water was used. Microcapsules of median diameter 329nm were produced with an encapsulation yield of 98%.

### Example 4

51.0g OMC was mixed with 19.0g avobenzone UV-A sunscreen oil and 0.400g dimethyltin dineodecanoate catalyst and emulsified in 90g water using 2.77g Laureth 3 and 6.20g Arquad 16-29. 30.0g TEOS was added and microcapsules were produced by the procedure described in Example 1. Microcapsules of median diameter 400nm were produced with a specific area of 17 m2/g.

### Example 5

0.2% xanthan gum was dispersed in 61.16% water at 60oC and mixed with 4% nonionic emulsifier and 2% glycerine. 10% cyclopentasiloxane was mixed with 4% capric/caprylic triglycerides and 0.5% preservative and heated to 60oC, then mixed with the above aqueous dispersion. The resulting emulsion was allowed to cool to 35oC and 17.14% of the suspension of microcapsules produced in Example 2 was added to form an oil in water sunscreen cream containing 6% OMC.

In vitro SPF measurements were performed on the above cream in comparison with a similar cream containing no microcapsules but containing 6% OMC mixed into the cyclopentasiloxane phase. The results are shown in Table 2.

**Table 2**

| | In-vitro SPF |
|---|---|
| O/w cream with non encapsulated OMC | 5.24 |
| O/w cream with encapsulated OMC of Example 2 | 10.18 |

As can be seen from Table 2, the encapsulated OMC gives a higher SPF than non-encapsulated OMC.

Sensory evaluation tests using paired comparison methods were conducted on the creams containing encapsulated and non encapsulated OMC. The results showed a positive impact of the encapsulated product on the speed of absorption of the cream.

### Example 6

17.0% of the suspension of microcapsules produced in Example 2 was diluted in 56.0% water containing 2.0% NaCl and then dispersed into 25.0% of a mixture of cyclomethicone and dimethicone copolyol slowly under strong agitation. The dispersion was passed through a homogeniser to produce a water in oil sunscreen cream.

Pigments can be added to the formulation of Example 6 or the formulation of Example 5 to make a foundation product with sun protection.

### Example 7

10.0% lauryl ether sulfate anionic surfactant was mixed with 2.5% decyl polyglucoside and 5.0% cocamidopropyl betaine. 2.0% Laureth-4 nonionic surfactant was added under strong agitation, followed by 7.5% of the encapsulated sunscreen suspension of Example 2, then 0.5% NaCl, citric acid and KOH to pH 6.5, preservatives and water to 100% to form a shower gel or shampoo which gives sun protection.

### Example 8

1.5% hydroxyethyl cellulose (HEC) was dispersed in 85.5% water and heated to 80 °C. 1.0 % cetearyl alcohol and 1.0 % of a mixture of polyethylene glycol (100) stearate and glyceryl Stearate were melted at 80 °C and the HEC dispersion was added under strong agitation. The resulting dispersion was allowed to cool down to 35 °C and 11.0% of the encapsulated sunscreen suspension of Example 2 was added, followed by preservative to from a rinse-off conditioner giving sun protection.

A leave-on conditioner with sun protection can be formed by simply diluting the encapsulated sunscreen suspension and adding preservative and perfume.

### Examples 9 and 10

The process of Example 2 was repeated with the omission of the nonionic surfactant and the reduction of the amount of Arquad 16-29 cationic surfactant to 1.83g (Example 9) and 0.915g (Example 10).

### Example 11

The process of Example 2 was repeated with the omission of the cationic surfactant.

### Example 12

The process of Example 11 was repeated with the amount of Volpo L3 nonionic surfactant being halved to 0.82g.

### Examples 13 and 14

The process of Example 1 was repeated with the omission of the nonionic surfactant and the reduction of the amount of Arquad 16-29 cationic surfactant to 0.915g (Example 13) or to 0.366g (Example 14).

The suspensions of microcapsules produced in Examples 9 to 13 were tested by the paraffinic solvent extraction method described above. The amount of OMC extracted after 24 hours is shown in Table 3 below

**Table 3**

| Example No | % extracted |
|---|---|
| 9 | 8 |
| 10 | 4 |
| 11 | 38 |
| 12 | 25 |
| 13 | 4 |
| 14 | 3 |

The test results of the microcapsule suspensions of Example 9, 10, 13 and 14 correspond to negligible leaching of the OMC from the microcapsules in a normal sunscreen vehicle. For the suspension of Example 14, the test was continued to 120 hours at which time 21% of the OMC was extracted.

### Example 15

Example 14 was repeated with an increase of the amount of TEOS added to 22.14g (25% increase). When the resulting suspension of microcapsules was tested by the paraffinic solvent extraction method described above, the amount of OMC extracted after 24 hours was 9% compared to 28% for the suspension of Example 14.

## Claims

1. A process for encapsulating a lipophilic cosmetic, chemical, biological or pharmaceutical active material composition, **characterised in that** a water reactive silicon compound comprising tetraalkoxysilane is added to an emulsion of the active material composition emulsified in an aqueous medium in the presence of a cationic or amphoteric surfactant, the emulsion having a positive zeta-potential, whereby the tetraalkoxysilane condenses and polymerises at the interface of the emulsified droplets of the lipophilic active material composition to form microcapsules having a core of the active material composition surrounded by a shell of silicon-based network polymer.

2. A process according to Claim 1, **characterised in that** the water reactive silicon compound comprises tetraethyl orthosilicate.

3. A process according to Claim 1 or Claim 2, **characterised in that** the concentration of surfactant in the emulsion is 0.02 to 2.0% by weight of the emulsion.

4. A process according to any of Claims 1 to 3, **characterised in that** a catalyst for hydrolysis and/or condensation of the water reactive silicon compound is added to the emulsion before, during or after the addition of the water-reactive silicon compound.

5. A process according to Claim 4, **characterised in that** the catalyst is an organic tin compound.

6. A process according to any of Claims 1 to 5, **characterised in that** the emulsion is passed through a high shear mixer after addition of the tetraalkoxysilane and before formation of microcapsules is complete.

7. A process according to any of Claims 1 to 6, **characterised in that** polyvinylpyrrolidone is added to the emulsion before addition of the tetraalkoxysilane.

8. A process according to any of Claims 1 to 7, **characterised in that** the active material is a liquid sunscreen composition.

9. A process according to any of Claims 1 to 8, **characterised in that** the microcapsules are separated from suspension by a liquid removal technique.

10. A method of producing a water based preparation comprising a lipophilic cosmetic, chemical, biological or pharmaceutical active material, **characterised in that** a suspension of encapsulated lipophilic cosmetic, chemical, biological or pharmaceutical active material composition is prepared according to any of Claims 1 to 8 and is incorporated direct into the water based preparation without separation of the microcapsules from the suspension.

11. A method for the preparation of a water based cosmetic preparation containing an encapsulated sunscreen, **characterised in that** a water based cosmetic preparation is mixed with at least one encapsulated sunscreen prepared according to Claim 8 in such a proportion that the content of encapsulated sunscreen in the toiletry preparation is 0.1 to 10% by weight.

12. An encapsulated cosmetic, biological or pharmaceutical active material composition, **characterised in that** the encapsulated composition comprises microcapsules of a lipophilic cosmetic, biological or pharmaceutical active material composition encapsulated within a shell of the emulsion polymerisation product of a tetraalkoxysilane, produced by the process of any of claims 1-8.

13. An encapsulated cosmetic, chemical, biological or pharmaceutical active material composition according to Claim 12, **characterised in that** the mean particle size of the microcapsules is between 200 nm and 10 µm.

## Patentansprüche

1. Ein Verfahren zum Verkapseln einer Zusammensetzung mit einer lipophilen kosmetisch, chemisch, biologisch oder pharmazeutisch aktiven Substanz, **dadurch gekennzeichnet, dass** eine mit Wasser reaktive Siliciumverbindung, die Tetraalkoxysilan enthält, zu einer Emulsion der Zusammensetzung mit aktiver Substanz, welche in einem wässrigen Medium in Gegenwart eines kationischen oder amphoteren oberflächenaktiven Mittels emulgiert ist, gegeben wird und die Emulsion ein positives Zeta-Potential hat, wobei das Tetraalkoxysilan kondensiert und an der Grenzfläche der emulgierten Tröpfchen der Zusammensetzung mit lipophiler aktiver Substanz polymerisiert, um Mikrokapseln zu bilden, die einen Kern der Zusammensetzung mit aktiver Substanz haben, umgeben von einer Schale eines Netzpolymers auf Siliciumbasis.

2. Ein Verfahren nach Anspruch 1, **dadurch gekennzeichnet, dass** die mit Wasser reaktive Siliciumverbindung Tetraethylorthosilicat enthält.

3. Ein Verfahren nach Anspruch 1 oder 2, **dadurch gekennzeichnet, dass** die Konzentration des oberflächenaktiven Mittels in der Emulsion 0,02 bis 2,0 Gew.-% der Emulsion beträgt.

4. Ein Verfahren nach einem der Ansprüche 1 bis 3, **dadurch gekennzeichnet, dass** ein Katalysator für die Hydrolyse und/oder Kondensation der mit Wasser reaktiven Siliciumverbindung zu der Emulsion vor, während oder nach der Zugabe der mit Wasser reaktiven Siliciumverbindung gegeben wird.

5. Ein Verfahren nach Anspruch 4, **dadurch gekennzeichnet, dass** der Katalysator eine organische Zinnverbindung ist.

6. Ein Verfahren nach einem der Ansprüche 1 bis 5, **dadurch gekennzeichnet, dass** die Emulsion durch einen Hochschermischer nach der Zugabe des Tetraalkoxysilans und bevor die Ausbildung der Mikrokapseln vollständig ist, geführt wird.

7. Ein Verfahren nach einem der Ansprüche 1 bis 6, **dadurch gekennzeichnet, dass** Polyvinylpyrrolidon zu der Emulsion vor Zugabe des Tetraalkoxysilans gegeben wird.

8. Ein Verfahren nach einem der Ansprüche 1 bis 7, **dadurch gekennzeichnet, dass** die aktive Substanz eine flüssige Sonnenschutzzusammensetzung ist.

9. Ein Verfahren nach einem der Ansprüche 1 bis 8, **dadurch gekennzeichnet, dass** die Mikrokapseln von der Suspension durch eine Flüssigkeitsentfernungstechnik abgetrennt werden.

10. Ein Verfahren zur Herstellung einer Zubereitung auf Wasserbasis, enthaltend eine lipophile kosmetisch, chemisch, biologisch oder pharmazeutisch aktive Substanz, **dadurch gekennzeichnet, dass** eine Suspension einer verkapselten Zusammensetzung mit lipophiler kosmetisch, chemisch, biologisch oder pharmazeutisch aktiver Substanz nach einem der Ansprüche 1 bis 8 hergestellt wird und direkt in die Zubereitung auf Wasserbasis eingearbeitet wird, ohne dass die Mikrokapseln von der Suspension abgetrennt werden.

11. Ein Verfahren zur Herstellung einer kosmetischen Zubereitung auf Wasserbasis, die ein verkapseltes Sonnenschutzmittel enthält, **dadurch gekennzeichnet, dass** eine kosmetische Zubereitung auf Wasserbasis mit wenigstens einem verkapselten Sonnenschutzmittel, hergestellt nach Anspruch 8, in solch einem Verhältnis gemischt wird, dass der Gehalt des verkapselten Sonnenschutzmittels in der kosmetischen Zubereitung von 0,1 bis 10 Gew.-% beträgt.

12. Eine verkapselte Zusammensetzung mit kosmetisch, biologisch oder pharmazeutisch aktiver Substanz, **dadurch gekennzeichnet, dass** die verkapselte Zusammensetzung Mikrokapseln einer Zusammensetzung mit lipophiler kosmetisch, biologisch oder pharmazeutisch aktiver Substanz enthält, die in einer Schale des Emulsionspolymerisationsproduktes eines Tetraalkoxysilans verkapselt sind, hergestellt durch ein Verfahren nach einem der Ansprüche 1 bis 8.

13. Eine verkapselte Zusammensetzung mit kosmetisch, chemisch, biologisch oder pharmazeutisch aktiver Substanz nach Anspruch 12, **dadurch gekennzeichnet, dass** die mittlere Teilchengröße der Mikrokapseln zwischen 200 nm und 10 µm liegt.

## Revendications

1. Procédé pour encapsuler une composition de substance active cosmétique, chimique, biologique ou pharmaceutique lipophile, **caractérisé en ce qu'**un composé du silicium réactif avec l'eau comprenant un tétraalcoxysilane est ajouté à une émulsion de la composition de substance active émulsifiée dans un milieu aqueux en présence d'un tensioactif cationique ou amphotère, l'émulsion ayant un potentiel zêta positif, de sorte que le tétraalcoxysilane se condense et polymérise à l'interface des gouttelettes émulsifiées de la composition de substance active lipophile pour former des microcapsules ayant un noyau de la composition de substance active entouré par une enveloppe de polymère en réseau à base de silicium.

2. Procédé selon la revendication 1, **caractérisé en ce que** le composé du silicium réactif avec l'eau comprend de l'orthosilicate de tétraéthyle.

3. Procédé selon la revendication 1 ou la revendication 2, **caractérisé en ce que** la concentration de tensioactif dans l'émulsion est 0,02 à 2,0 % par rapport à la masse de l'émulsion.

4. Procédé selon l'une quelconque des revendications 1 à 3, **caractérisé en ce qu'**un catalyseur pour l'hydrolyse et/ou la condensation du composé du silicium réactif avec l'eau est ajouté à l'émulsion avant, pendant ou après l'addition du composé du silicium réactif avec l'eau.

5. Procédé selon la revendication 4, **caractérisé en ce que** le catalyseur est un composé organique de l'étain.

6. Procédé selon l'une quelconque des revendications 1 à 5, **caractérisé en ce que** l'émulsion est amenée à traverser un mélangeur à haut cisaillement après l'addition du tétraalcoxysilane et avant que la formation de microcapsules soit achevée.

7. Procédé selon l'une quelconque des revendications 1 à 6, **caractérisé en ce que** de la polyvinylpyrrolidone est ajoutée à l'émulsion avant l'addition du tétraalcoxysilane.

8. Procédé selon l'une quelconque des revendications 1 à 7, **caractérisé en ce que** la substance active est une composition de filtre solaire liquide.

9. Procédé selon l'une quelconque des revendications 1 à 8, **caractérisé en ce que** les microcapsules sont séparées de la suspension par une technique de retrait de liquide.

10. Procédé de production d'une préparation à base aqueuse comprenant une substance active cosmétique, chimique, biologique ou pharmaceutique lipophile, **caractérisé en ce qu'**une suspension de composition de substance active cosmétique, chimique, biologique ou pharmaceutique lipophile encapsulée est préparée selon l'une quelconque des revendications 1 à 8 et est incorporée directement dans la préparation à base aqueuse sans séparation des microcapsules de la suspension.

11. Procédé pour la préparation d'une préparation cosmétique à base aqueuse contenant un filtre solaire encapsulé, **caractérisé en ce qu'**une préparation cosmétique à base aqueuse est mélangée avec au moins un filtre solaire encapsulé préparé selon la revendication 8 dans une proportion telle que la teneur du filtre solaire encapsulé dans la préparation de type article de toilette est 0,1 à 10 % en masse.

12. Composition de substance active cosmétique, biologique ou pharmaceutique encapsulée, **caractérisée en ce que** la composition encapsulée comprend des microcapsules d'une composition de substance active cosmétique, biologique ou pharmaceutique lipophile encapsulée dans une enveloppe du produit de polymérisation en émulsion d'un tétraalcoxysilane produit par le procédé selon l'une quelconque des revendications 1 à 8.

13. Composition de substance active cosmétique, chimique, biologique ou pharmaceutique encapsulée selon la revendication 12, **caractérisée en ce que** la taille de particule moyenne des microcapsules est entre 200 nm et 10 µm.
